# EUROPÄISCHE PATENTSCHRIFT

(11) **EP 0 819 762 B1**
(45) Veröffentlichungstag und Bekanntmachung des Hinweises auf die Patenterteilung: **31.05.2006**
(21) Anmeldenummer: 97112306.2
(22) Anmeldetag: 17.07.1997
(51) Int. Cl.: C12P 19/04, C08L 5/00

(54) **Verfahren zur Isolierung von Glucan aus Hefe**
Process for the isolation of glucan from yeast
Procédé d'isolement de glucane à partir de levure

(30) Priorität: 19.07.1996 DE 19629118
(43) Veröffentlichungstag der Anmeldung: 21.01.1998
(73) Patentinhaber: Mibelle AG Cosmetics, 5033 Buchs (CH)
(72) Erfinder: Zülli, Fred, Dr. sc. nat., 5024 Küttigen (CH); Suter, Franz, 5312 Döttingen (CH)
(74) Vertreter: Lau-Loskill, Philipp

(56) Entgegenhaltungen:
- WO-A-94/03500
- GB-A- 802 526
- US-A- 4 810 646
- PATENT ABSTRACTS OF JAPAN vol. 1999, no. 08, 30. Juni 1999 (1999-06-30) & JP 11 056384 A (TOSOH CORP), 2. März 1999 (1999-03-02)

## Beschreibung

Die vorliegende Erfindung betrifft ein Verfahren zur Isolierung von Glucan aus Hefe mit den Merkmalen des Oberbegriffs des Patentanspruchs 1.

Um Glucan, das durch die nachfolgend wiedergegebene Formel I chemisch gekennzeichnet ist, 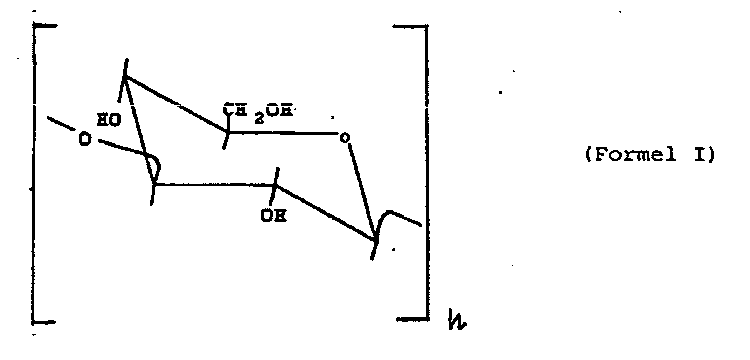 herzustellen, ist es bekannt, dieses Glucan aus Hefe zu isolieren.

So beschreibt beispielsweise die US 5,223,491 ein derartiges Verfahren, wobei bei dem bekannten Verfahren Hefe mit einer wäßrigen alkalischen Lösung unter Überdruck während 15 Minuten bis 1,5 Stunden aufgeschlossen wird. Anschließend wird der hierbei anfallende Rückstand nach Waschen mit Wasser in einer säurehaltigen Lösung während 30 Minuten bis etwa 3 Stunden bei 85 °C suspendiert. Hiernach erfolgt eine Abtrennung des in der wäßrigen Säure unlöslichen Rückstandes, der wiederum nach Trocknung mit absolutem Ethanol oder Aceton gewaschen wird.

Desweiteren beschreibt die WO 94/03500 ein Verfahren zur Herstellung von Glucan, wobei bei dem bekannten mehrstufigen Verfahren die Hefe zur Zerstörung der Zellen in einem Autoklav bei 121 °C mit einem Citratpuffer bei einem pH-Wert von 5 behandelt wird, hiernach sich eine Extraktion bei mindestens 100 °C mit wäßriger Natronlaufe und danach eine Extraktion mit verdünnten organischen Säuren für jeweils 24 Stunden anschließt. Hierauf folgen dann eine Behandlung des so isolierten Glucans mit einer wäßrigen Tensidlösung bei einem pH-Wert von 6,8 und anschließend ein mehrfaches waschen des Glucans mit Wasser.

Ein Verfahren mit den Merkmalen des Oberbegriffs des Patentanspruchs 1 ist aus der US 4,810,646 bekannt. Zur Isolierung des Glucans sieht das bekannte Verfahren vor, Hefezellen mit einer 4 %igen Natronlauge zunächst bei 100 °C zu behandeln, hiernach den Rückstand mit 3 %iger Natronlauge zu extrahieren und danach den erneut isolierten Rückstand mit Salzsäure bei einem pH-Wert von 4,5 und einer Temperatur von 75 °C zu behandeln. Anschließend wird das abzentrifugierte Produkt mit Wasser, absolutem Alkohol und zweifach mit absolutem Ether gewaschen, wobei das so hergestellte Glucan noch proteinhaltige Verunreinigungen aufweist.

Bedingt dadurch, daß bei den zuvor beschriebenen bekannten Isolierungsverfahren die Hefe zumindestens in der ersten Behandlungsstufe unter recht drastischen Temperatur- und Druckbedingungen mit einer 0,5-normalen bis 3-normalen Natronlauge aufgeschlossen wird, besteht die Gefahr, daß unter diesen drastischen Bedingungen eine unerwünschte chemische Veränderung des Glucans eintritt, die sich beispielsweise in einer Verfärbung des ansonsten weißen Glucans ausdrückt.

Der vorliegenden Erfindung liegt die Aufgabe zugrunde, ein Verfahren zur Isolierung von Glucan aus Hefe zur Verfügung zu stellen, das eine besonders schonende Arbeitsweise vorsieht.

Diese Aufgabe wird erfindungsgemäß durch ein Verfahren mit den kennzeichnenden Merkmalen des Patentanspruchs 1 gelöst.

Das erfindungsgemäße Verfahren zur Isolierung von Glucan aus Hefe schlägt vor, daß die Hefe mit einer alkalischen, tensidhaltigen Lösung behandelt wird. Der hierbei im Anschluß an die Behandlung mit der alkalischen, tensidhaltigen Lösung bei dem erfindungsgemäßen Verfahren anfallende feste Glucan-haltige Rückstand wird nach Abtrennung von der alkalischen, tensidhaltigen Lösung mit einem Alkohol in Kontakt gebracht. Bedingt dadurch, daß Glucan in dem eingesetzten Alkohol, bei dem es sich um einen C₁-C₄-Alkohol handelt, nicht löslich ist, kann das unlösliche Glucan hiernach aus der alkoholischen Aufschlämmung abgeschieden und hiernach getrocknet werden. Bei dem erfindungsgemäßen Verfahren wird als alkalische, tensidhaltige Lösung eine solche wasserhaltige Lösung ausgewählt, die außer dem mindestens einen Alkalihydroxid und/oder Erdalkalihydroxid 0,05 Gew.% bis 3 Gew.% eines Tensids oder Tensidgemisches enthält.

Überraschend konnte durch Anwendung des erfindungsgemäßen Verfahrens festgestellt werden, daß das Glucan mit einer besonders hohen Ausbeute und einer extremen Reinheit aus Hefe isoliert werden kann, wobei dieser überraschende Effekt darauf zurückgeführt wird, daß die aus dem zuvor beschriebenen bekannten Verfahren eingesetzte alkalische Lösung durch Zusatz eines Tensids in den zuvor angegebenen Konzentrationen bei dem erfindungsgemäßen Verfahren offensichtlich einen synergistischen Effekt in bezug auf die Isolierung des Glucans aus Hefe besitzt. Dies wiederum führt dazu, daß bei dem erfindungsgemäßen Verfahren auf die zuvor beim bekannten Verfahren beschriebene Säurebehandlung vollständig verzichtet werden kann, was einerseits den Vorteil hat, daß das erfindungsgemäße Verfahren wesentlich einfacherer und somit wirtschaftlicher durchzuführen ist und andererseits bei dem erfindungsgemäßen Verfahren eine Säureschädigung des Glucans, so zum Beispiel eine Säurehydrolyse, vermieden wird. Dies wiederum bewirkt, daß bei dem erfindungsgemäßen Verfahren ein alterungsstabiles, rein weißes Glucan von hohem Reinheitsgrad isoliert werden kann, so daß ein derartig isoliertes Glucan insbesondere auch im pharmazeutischen und/oder kosmetischen Bereich direkt oder in Form von entsprechenden Glucan-Derivaten, vorzugsweise von Carboxymethyl-Glucan oder anderen Glucanäthern, verwendbar ist.

Unter dem Begriff Hefe im Sinne der vorliegenden Anmeldung fallen alle an sich bekannten Hefezellen, so zum Beispiel Zellen aus dem Stämmen Saccharomyces cerevisiae, Saccharomyces delbrueckii, Saccharomyces rosei, Saccharomyces microellipsodes, Saccharomyces carlsbergensis, Saccharomyces bisporus, Saccharomyces fermentati, Saccharomyces rouxii, Schizosaccharomyces pombe, Kluyveromyces polysporus, Candida albicans, Candida cloacae, Candida tropicalis, Candida utilis, Hansenula wingei, Hansenula arni, Hansenula henricii, Hansenula americana, Hansenula canadiensis, Hansenula capsulata, Hansenula polymorpha, Pichia kluyveri, Pichia pastoris, Pichia polymorpha, Pichia rhodanensis, Pichia ohmeri, Torulopsis bovina, Torulopsis glabrata und insbesondere Bäckerhefe.

Um bei dem erfindungsgemäßen Verfahren eine besonders schonende Isolierung des Glucans aus Hefe sicherzustellen, empfiehlt es sich, die Konzentration des Alkalihydroxids und/oder des Erdalkalihydroxids in der alkalischen, tensidhaltigen Lösung relativ niedrig einzustellen, wobei vorzugsweise die Konzentration des Alkalihydroxids und/oder des Erdalkalihydroxids zwischen 5 Gew.% und 0,05 Gew.%, insbesondere zwischen 2 Gew.% und 0,1 Gew.%, in der alkalischen, tensidhaltigen Lösung variiert.

Grundsätzlich bestehen bei dem erfindungsgemäßen Verfahren zwei Möglichkeiten, die Isolierung des Glucans aus Hefe durchzuführen. So sieht die erste Möglichkeit vor, daß die Konzentration an Alkalihydroxid und/oder an Erdalkalihydroxid während der gesamten Isolierung konstant gehalten wird, wobei die Konzentration an Alkalihydroxid bzw. an Erdalkalihydroxid in den zuvor genannten Konzentrationsbereichen variieren.

Bei der zweiten, besonders geeigneten Ausführungsvariante des erfindungsgemäßen Verfahrens wird die Hefe zunächst mit einer bezüglich der Alkalihydroxid- und/oder Erdalkalihydroxid-konzentration höher konzentrierten alkalischen, tensidhaltigen Lösung behandelt, wodurch ein rasches, aber dennoch schonendes Aufschließen der Hefezellen herbeigeführt wird. Im Anschluß an diese Behandlung erfolgt dann ein weiterer Aufschluß der Hefe zur Isolierung des Glucans, wobei jedoch in dieser weiteren Behandlung die Alkalihydroxid- und/oder Erdalkalihydroxid-konzentration im Vergleich zur ersten Behandlung verringert ist.

Grundsätzlich kann bei dem erfindungsgemäßen Verfahren jedes beliebige Tensid in der alkalischen, tensidhaltigen Lösung enthalten sein, sofern sichergestellt ist, daß dieses Tensid einerseits den Aufschluß der Hefezellen und somit die Isolierung des Glucans beschleunigt und andererseits dieses Tensid unter den Bedingungen der Glucan-Isolierung stabil ist. So ist es beispielsweise möglich, als Tenside die an sich bekannten kationischen und/oder amphoteren Tenside einzusetzen. Besonders geeignet ist es jedoch, wenn bei dem erfindungsgemäßen Verfahren die alkalische, tensidhaltige Lösung als Tensid ein anionisches und/oder ein nichtionisches Tensid enthält. vorzugsweise weist jedoch bei dem erfindungsgemäßen Verfahren die alkalische, tensidhaltige Lösung als Tensid ein Alkalisalz eines Alkylsulfates und/oder ein Alkalisalz eines Alkylsulfonates auf.

Besonders gute Ergebnisse bezüglich der Ausbeute an Glucan und dessen Reinheit lassen sich dann erzielen, wenn bei dem erfindungsgemäßen Verfahren in der alkalischen, tensidhaltigen Lösung ein Natriumlaurylsulfonat in den eingangs angegebenen Konzentrationen eingesetzt wird. Hier konnte festgestellt werden, daß dieses Natriumlaurylsulfat einerseits den Aufschluß der Hefezellen beschleunigt und desweiteren die in der Hefe enthaltenen fettartigen Verbindungen bzw. Fette hervorragend emulgiert, so daß durch Verwendung von Natriumlaurylsulfat in der alkalischen, tensidhaltigen Lösung ein hochreines Glucan mit guter Ausbeute isoliert werden kann.

Um bei dem erfindungsgemäßen Verfahren desweiteren eine schonende Isolierung des Glucans zu erreichen, wird vorzugsweise ein Massenverhältnis von Hefe zur alkalischen, tensidhaltigen Lösung zwischen 1:10 und 1:400, insbesondere zwischen 1:20 und 1:200, eingestellt.

Eine besonders geeignete und vorteilhafte Ausführungsform des erfindungsgemäßen Verfahrens sieht vor, daß die Hefe während eines ersten Behandlungsschrittes mit der alkalischen, tensidhaltigen Lösung bei einer maximalen Temperatur von 30 °C behandelt wird. An diesen ersten Behandlungsschritt schließt sich dann ein zweiter Behandlungsschritt an, wobei die Temperatur der alkalischen, tensidhaltigen Lösung auf 50 °C bis 95 °C eingestellt wird.

Bei einer anderen, besonders geeigneten Weiterbildung der zuvor beschriebenen Ausführungsform des erfindungsgemäßen Verfahrens wird der erste Behandlungsschritt in zwei bis acht erste Behandlungsteilschritte aufgeteilt, wobei in jedem Behandlungsteilschritt ein fester, Glucan-haltiger Rückstand abgetrennt wird. Dieser abgetrennte, Glucan-haltige Rückstand wird dann in jedem Behandlungsteilschritt mit frischer alkalischer, tensidhaltiger Lösung jeweils in Kontakt gebracht, wobei die hierfür eingesetzte alkalische, tensidhaltige Lösung wahlweise eine gleichbleibende Konzentration an Alkalihydroxid und/oder Erdalkalihydroxid oder eine unterschiedliche Konzentration, vorzugsweise eine abnehmende Konzentration, an Alkalihydroxid und/oder Erdalkalihydroxid aufweist. So konnte beispielsweise festgestellt werden, daß besonders reines Glucan dadurch aus Hefe isoliert wird, wenn im ersten Behandlungsteilschritt des ersten Behandlungsschrittes eine Konzentration an Alkalihydroxid und/oder Erdalkalihydroxid in der alkalischen, tensidhaltigen Lösung zwischen etwa 2 Gew.% und 1 Gew.% eingestellt wird. Anschließend werden dann in den folgenden Behandlungsteilschritten des ersten Behandlungsschrittes die jeweils abgetrennten Rückstände mit alkalischen, tensidhaltigen Lösungen behandelt, deren Konzentrationen an Alkalihydroxid und/oder Erdalkalihydroxid deutlich unter der Alkalihydroxid- bzw. Erdalkalihydroxid-Konzentration des ersten Behandlungsteilschrittes liegen, wie dies nachfolgend an einem konkreten Ausführungsbeispiel noch im Detail erläutert ist.

Wird bei dem erfindungsgemäßen Verfahren nach Durchführung eines jeden ersten Behandlungsteilschrittes die alkalische, tensidhaltige Lösung ausgetauscht, so variiert das Massenverhältnis von abgetrenntem Glucan-haltigem Rückstand und alkalischer, tensidhaltiger Lösung zwischen 1:1 und 1:40.

Eine Weiterbildung der zuvor beschriebenen Ausführungsvariante des erfindungsgemäßen Verfahrens sieht vor, daß auch der zweite Behandlungsschritt in zwei bis acht zweite Behandlungsteilschritte aufgeteilt wird, wobei in jedem Behandlungsteilschritt ein fester, Glucan-haltiger Rückstand abgetrennt wird, so daß dann dieser abgetrennte Rückstand jeweils mit frischer alkalischer, tensidhaltiger Lösung in Kontakt gebracht wird.

Insbesondere dann, wenn bei der Durchführung des zweiten Behandlungsschrittes bzw. der zweiten Behandlungsteilschritte die Konzentration an Alkalihydroxid und/oder Erdalkalihydroxid in der alkalischen, tensidhaltigen Lösung im Vergleich zur Alkalihydroxid- bzw. Erdalkalihydroxid-Konzentration im ersten Behandlungsschritt bzw. in den ersten Behandlungsteilschritten reduziert wird, ermöglicht dieser Verfahrensvariante eine besonders schonende Isolierung des Glucans.

Bezüglich der Behandlungszeiten, die bei dem erfindungsgemäßen Verfahren zur Isolierung des Glucans aus Hefe erforderlich sind, ist festzuhalten, daß sich diese Behandlungszeiten allgemein danach richten, welche der zuvor beschriebenen Verfahrensvarianten durchgeführt werden. Üblicherweise variieren diese Behandlungszeiten zwischen etwa 30 Stunden und etwa 480 Stunden, wobei die zuletzt genannten langen Behandlungszeiten dann anzuwenden sind, wenn das erfindungsgemäße Verfahren einen ersten und einen zweiten Behandlungsschritt vorsieht, die jeweils in zwei bis acht Behandlungsteilschritte aufgeteilt werden. Hierbei variiert dann die Behandlungszeit in jedem einzelnen Behandlungsteilschritt zwischen 5 Stunden und 48 Stunden.

Um bei dem erfindungsgemäßen Verfahren das nach der Behandlung mit der alkalischen, tensidhaltigen Lösung anfallende Glucan in Reinform zu isolieren, wird der unlösliche, Glucan-haltige Rückstand aus der alkalischen, tensidhaltigen Lösung zunächst abgetrennt. Der abgetrennte Rückstand wird dann mit Wasser, ggf. unter Zusatz eines Tensids, gewaschen. Der so mit Wasser gewaschene Rückstand wird hiernach mit dem C₁-C₄-Alkohol in Kontakt gebracht, wobei durch diese Behandlung fettartige Verbindungen bzw. Fette in Lösung gebracht werden, so daß nach der Alkoholbehandlung dann das in dem entsprechenden C₁-C₄₋Alkohol unlösliche gereinigte Glucan abgetrennt wird.

Um bei dem erfindungsgemäßen Verfahren eine restlose Abtrennung von Fetten, Lipide und fettartigen Verbindungen sicherzustellen, sieht eine weitere Ausführungsform des erfindungsgemäßen Verfahrens vor, daß hierbei der in Wasser gewaschene, Glucan-haltige Rückstand zweimal bis sechsmal mit dem C₁-C₄₋Alkohol in Kontakt gebracht wird, wobei vorzugsweise nach jedem in Kontakt bringen der in Alkohol unlösliche Glucan-haltige Rückstand abgetrennt und erneut mit frischem C₁-C₄₋Alkohol wieder versetzt wird.

Insbesondere dann, wenn jedes in Kontakt bringen des festen, Glucan-haltigen Rückstands mit dem C₁-C₄-Alkohol für 5 Stunden bis 48 Stunden bei einer Temperatur zwischen 20 °C und 60 °C durchgeführt wird, läßt sich bei dem erfindungsgemäßen Verfahren durch diese Verfahrensführung ein hochreines Glucan isolieren, in dem keine Fette, Lipide oder sonstige fettartige Verbindungen mehr enthalten sind.

Bezüglich des beim erfindungsgemäßen Verfahren eingesetzten Alkohols ist nochmals festzuhalten, daß hierfür die niedrigen C₁-C₄-Alkohole und vorzugsweise Isopropanol ausgewählt werden bzw. wird.

Das nach dem erfindungsgemäßen Verfahren isolierte Glucan wird insbesondere direkt in pharmazeutischen und/oder kosmetischen Produkten verwendet. Hier konnte festgestellt werden, daß ein derartige hochreines und nach dem erfindungsgemäßen Verfahren isoliertes Glucan, dessen Molekulargewicht vorzugsweise zwischen 60.000 und 150.000 variiert, hervorragend zur Behandlung und Prophylaxe von Hauterkrankungen einsetzbar ist, insbesondere solchen Hauterkrankungen, die durch oxidativen Streß in der Haut hervorgerufen werden. Hier ist insbesondere die Alterung der Haut, die Beeinträchtigung der Haut durch UVA-Strahlen und/oder durch oxidativen Chemikalien zu nennen.

Desweiteren läßt sich das nach dem erfindungsgemäßen Verfahren isolierte Glucan zur Herstellung von in pharmazeutischen und/oder kosmetischen Zubereitungen einsetzbaren Glucan-Derivaten verwenden, wobei als Glucan-Derivat hier insbesondere Carboxymethyl-Glucan oder Glucanäther zu nennen ist. Dieses Carboxymethyl-Glucan besitzt ebenfalls hervorragende prophylaktische bzw. therapeutische Eigenschaften in bezug auf die zuvor aufgeführten Hauterkrankungen, wobei diese therapeutischen bzw. prophylaktischen Eigenschaften des nach dem erfindungsgemäßen Verfahren isolierten Glucans und der daraus hergestellten Derivate deutlich überlegen sind im Vergleich zu solchen Glucanen, die nach herkömmlichen Verfahren isoliert worden sind. Dies hängt vermutlich damit zusammen, daß durch die zuvor beschriebene extrem schonende Isolierung des Glucans im linearen 1,3-beta-Glucan eine optimale Menge von 1,6 Verzweigungspunkten erhalten bleibt, die bei der anschließenden Derivatisierung zu reproduzierbaren und einheitlichen Derivatisierungsprodukten führen.

Im Rahmen des vorliegenden Textes wird unter Alkalihydroxid insbesondere Natriumhydroxid oder Kaliumhydroxid und unter Erdalkalihydroxid vorzugsweise Calciumhydroxid verstanden, wobei Natriumhydroxid bei dem erfindungsgemäßen Verfahren bevorzugt wird.

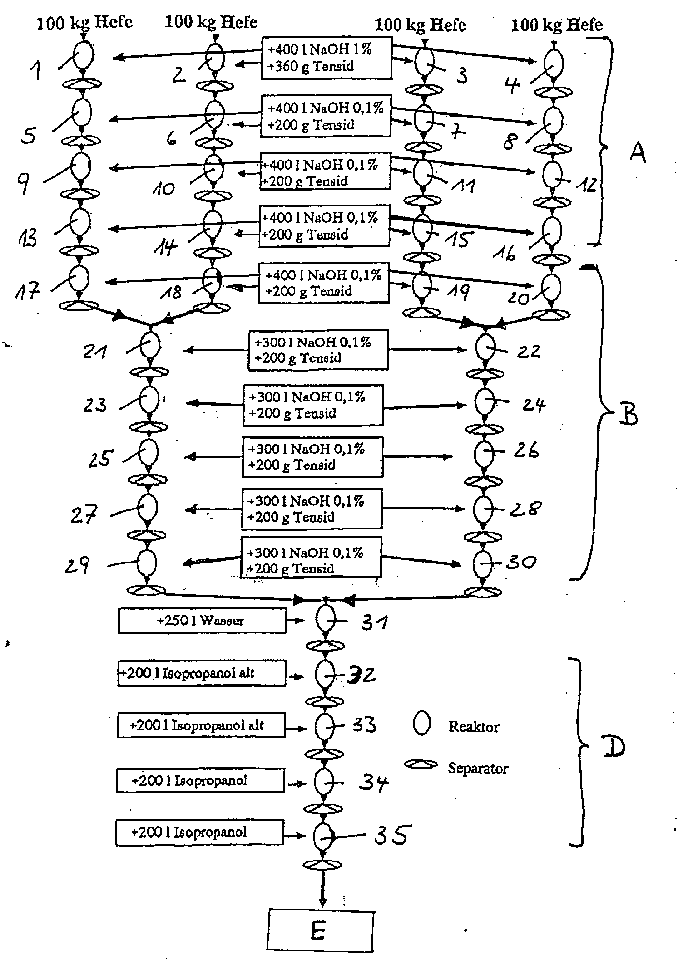

Das erfindungsgemäße Verfahren wird nachfolgend anhand eines Ausführungsbeispiels in Verbindung mit der Zeichnung näher erläutert. Hierbei zeigt die einzige Figur der Zeichnung ein Fließschema des erfindungsgemäßen Verfahrens.

In vier Reaktoren 1 - 4 wurden jeweils 100 kg Bäckerhefe vorgelegt. Anschließend wurde in jedem der Reaktoren 1 - 4 jeweils 400 1 einer alkalischen, wäßrigen, tensidhaltigen Lösung, bestehend aus 1 Gew.% Natriumhydroxid (fest) und 360 g Natriumlaurylsulfat, zugegeben.

Nach einem ersten Behandlungsteilschritt, der bei Raumtemperatur (16 °C - 20 °C) während 10 Stunden durchgeführt wurde, wurde der unlösliche, Glucan-haltige Rückstand mittels Separatoren von der flüssigen alkalischen und tensidhaltigen Lösung abgetrennt.

Die so gewonnenen Rückstände wurden in die Reaktoren 5 - 8 überführt. Dort wurden die Rückstände jeweils mit 400 1 einer alkalischen Lösung, bestehend aus 0,1 Gew.% Natriumhydroxid (fest) und 200 g des zuvor genannten Tensids versetzt und während 10 Stunden bei Raumtemperatur (16 °C - 20 °C) behandelt. Nach Abschluß dieses weiteren Behandlungsteilschrittes wurde der feste, Glucan-haltige Rückstand von der alkalischen, tensidhaltigen Lösung abgetrennt.

Dieser feste Rückstand wurde jeweils in die Reaktoren 9 - 12 überführt. In den Reaktoren 9 - 12 wurde der feste, Glucan-haltige Rückstand mit jeweils 400 l einer alkalischen Lösung, bestehend aus 0,1 Gew.% Natriumhydroxid (fest) und 200 g des zuvor genannten Tensids, versetzt. Nach einer Behandlungszeit von 10 Stunden bei Raumtemperatur (16 °C - 20 °C) wurde der in den Reaktoren 9 - 12 anfallende Rückstand von der alkalischen Lösung separiert und in die Reaktoren 13 - 16 überführt.

In den Reaktoren 13 - 16 wurde der feste, Glucan-haltige Rückstand mit 400 1 einer alkalischen Lösung, bestehend aus 0,1 Gew.% Natriumhydroxid (fest) und 200 g des zuvor genannten Tensids, versetzt und für 10 Stunden bei Raumtemperatur (16 °C - 20 °C) mit der zuvor genannten alkalischen Lösung in einem weiteren Behandlungsteilschritt behandelt. Hierbei fiel am Ende dieses Behandlungsteilschrittes der feste, Glucan-haltige Rückstand an, der über die entsprechenden Separatoren abgetrennt wurde.

Die vorstehend beschriebenen Behandlungsteilschritte bildeten den ersten Behandlungsschritt A aus.

Nach Abschluß des ersten Behandlungsschrittes A wurde der feste, Glucan-haltige Rückstand in die Reaktoren 17 - 20 überführt. In diesen Reaktoren 17 - 20 wurde dieser feste, Glucan-haltige Rückstand jeweils mit 400 1 alkalischer Lösung, bestehend aus 0,1 Gew.% Natriumhydroxid (fest) und 200 g des zuvor genannten Tensids, behandelt, wobei jedoch in diesem Behandlungsteilschritt des zweiten Behandlungsschrittes B die Temperatur 80 °C und die Verweilzeit 10 Stunden betrug.

Nach Ablauf dieser Behandlungszeit wurde der feste, Glucan-haltige Rückstand der Reaktoren 17 und 18 in den Reaktor 21 und der feste, Glucan-haltige Rückstand der Reaktoren 19 und 20 in den Reaktor 22 überführt, wobei sowohl zu dem Reaktor 21 als auch zu dem Reaktor 22 jeweils 300 l einer alkalischen Lösung, bestehend aus 0,1 Gew.% Natriumhydroxid (fest) und 200 g des zuvor genannten Tensids, zugegeben wurden. In den Reaktoren 21 und 22 wurde der feste, Glucan-haltige Rückstand während 10 Stunden bei 80 °C mit den zuvor beschriebenen alkalischen Lösungen behandelt.

Nach Ablauf der zuvor genannten Reaktionszeit wurden die Rückstände aus den Reaktoren 21 und 22 von der alkalischen Lösung abgetrennt und in die Reaktoren 23 und 24 überführt. Jeder Reaktor 23 bzw. 24 wurde hiernach mit 300 l alkalischer Lösung, bestehend aus 0,1 Gew.% Natriumhydroxid (fest) und 200 g des zuvor genannten Tensids, befüllt. Nach Ablauf einer Reaktionszeit von 10 Stunden bei 80 °C wurden die festen, Glucan-haltigen Rückstände von der alkalischen Lösungen abgetrennt und in die Reaktoren 25 und 26 überführt.

In den Reaktoren 25 bzw. 26 wurden die festen Rückstände jeweils mit 300 l einer alkalischen Lösung, enthaltend 0,1 Gew.% Natriumhydroxid (fest) und 200 g des zuvor genannten Tensids, behandelt. Nach Ablauf der Behandlungszeit von 10 Stunden bei einer Behandlungstemperatur von 80 °C wurden die festen, Glucan-haltigen Rückstände abgetrennt und in die Reaktoren 27 und 28 überführt.

In den Reaktoren 27 und 28 wurden jeweils 300 l einer alkalischen Lösung, bestehend aus 0,1 Gew.% Natriumhydroxid (fest) und 200 g des zuvor genannten Tensids, eingespeist, so daß die festen (unlöslichen), Glucan-haltige Rückstände in den Reaktoren 27 und 28 während 10 Stunden bei 80 °C behandelt werden konnten.

Nach Abschluß dieses Behandlungsteilschrittes wurden die festen, Glucan-haltigen Rückstände abgetrennt und in die Reaktoren 29 und 30 überführt. In den Reaktoren 29 und 30 wurden die festen, Glucan-haltigen Rückstände mit jeweils 300 l einer alkalischen Lösung, enthaltend 0,1 Gew.% Natriumhydroxid (fest) und 200 g des zuvor genannten Tensids, während 10 Stunden bei 80 °C behandelt. Hiernach wurden die in den Reaktoren 29 und 30 anfallenden festen, Glucan-haltige Rückstände vereinigt und in den Reaktor 31 überführt.

Die zuvor beschriebenen Behandlungsteilschritte, die in den Reaktoren 17 bis 30 bei 80 °C durchgeführt wurden, stellen den zweiten Behandlungsschritt B dar.

Nachdem die festen, Glucan-haltigen Rückstände aus den Reaktoren 29 und 30 in dem Reaktor 31 vereinigt wurden, wurde der so vereinigte Rückstand in dem Reaktor 31 während 10 Stunden mit 250 l Wasser bei 50 °C behandelt. Nach Abschluß dieser Behandlung wurde der unlösliche, Glucan-haltige Rückstand von dem Wasser abgetrennt und in den Reaktor 32 überführt.

Im Reaktor 32 wurde der feste, Glucan-haltige Rückstand zunächst mit 200 l Isopropanol versetzt und dort 10 Stunden bei 50 °C behandelt.

Nach Ablauf der Behandlungszeit von 10 Stunden wurde der in Isopropanol unlösliche, Glucan-haltige Rückstand in den Reaktor 33 überführt und dort während 10 Stunden bei 50 °C mit 200 l Isopropanol behandelt.

Nach Ablauf dieser Behandlung im Reaktor 33 wurde der feste, Glucan-haltige Rückstand abgetrennt und in den Reaktor 34 überführt, wobei im Reaktor 34 der feste, Glucan-haltige Rückstand mit 200 l frischem Isopropanol versetzt wurde. Nach Ablauf einer Behandlungszeit von 10 Stunden bei 50 °C wurde der feste, Glucan-haltige Rückstand vom Isopropanol abgetrennt und in den Reaktor 35 überführt. Das hierbei aus dem Reaktor 34 abgezogene Isopropanol wurde in einen Vorratstank überführt, so daß es für die Behandlung einer nächsten Charge Glucan-haltiger Rückstände in den Reaktor 32 oder 33 wieder eingespeist werden konnte, wobei in der Figur dieses Isopropanol mit Isopropanol "alt" bezeichnet ist.

Im Reaktor 35 wurde der feste, Glucan-haltige Rückstand mit 200 1 frischem Isopropanol während 10 Stunden bei 50 °C behandelt, wobei nach Ablauf dieser Behandlung das so isolierte und gereinigte Glucan separiert und einer Trocknungsstufe E unterworfen wurde. Das am Ende der Behandlung im Reaktor 35 anfallende Isopropanol wurde in einen Vorratstank gepumpt und dort aufbewahrt, so daß es für eine nächste Charge Glucan-haltiger Rückstände in den Reaktor 33 oder 32 eingespeist werden kann, wobei in der Figur dieses Isopropanol mit Isopropanol "alt" bezeichnet ist.

Die zuvor beschriebenen und in den Reaktoren 32 bis 35 ablaufenden Behandlungsstufen mit Isopropanol sind auch insgesamt in der Figur als Behandlungsschritt D zusammengefaßt.

Die Ausbeute an hochreinem Glucan betrug 8 kg = 2 Gew.% der eingesetzten Hefe.

## Patentansprüche

1. Verfahren zur Isolierung von Glucan aus Hefe, bei dem die Hefe mit einer wäßrigen, alkalischen Lösung behandelt wird, der hierbei anfallende feste Glucan-haltige Rückstand mit einem Alkohol in Kontakt gebracht und anschließend das so isolierte Glucan getrocknet wird, **dadurch gekennzeichnet, daß** als alkalische Lösung eine alkalische, tensidhaltige Lösung ausgewählt wird, die außer einem Alkalihydroxid und/oder Erdalkalihydroxid ein Tensid oder ein Tensidgemisch enthält, wobei die Konzentration des Tensids bzw. Tensidgemisches zwischen 0,05 Gew.% und 3 Gew.% liegt, und daß der Glucan-haltige Rückstand mit einem C₁-C₄-Alkohol in Kontakt gebracht wird.

2. Verfahren nach Anspruch 1, **dadurch gekennzeichnet, daß** die alkalische, tensidhaltige Lösung zwischen 5 Gew.% und 0,05 Gew.%, vorzugsweise zwischen 2 Gew.% und 0,1 Gew.%, des Alkalihydroxids und/oder des Erdalkalihydroxids enthält.

3. Verfahren nach Anspruch 1 oder 2, **dadurch gekennzeichnet, daß** die Hefe zunächst mit einer bezüglich der Alkalihydroxid- und/oder Erdalkalihydroxid-Konzentration höher konzentrierten alkalischen, tensidhaltigen Lösung behandelt wird und daß hiernach die Alkalihydroxid- und/oder Erdalkalihydroxid-Konzentration im Laufe der Isolierung des Glucans verringert wird.

4. Verfahren nach einem der vorangehenden Ansprüche, **dadurch gekennzeichnet, daß** die alkalische, tensidhaltige Lösung als Tensid ein anionisches und/oder ein nichtionisches Tensid enthält.

5. Verfahren nach Anspruch 4, **dadurch gekennzeichnet, daß** die alkalische, tensidhaltige Lösung als Tensid ein Alkalisalz eines Alkylsulfates und/oder ein Alkalisalz eines Alkylsulfonates aufweist.

6. Verfahren nach Anspruch 4 oder 5, **dadurch gekennzeichnet, daß** die alkalische, tensidhaltige Lösung als Tensid ein Natriumlaurylsulfat enthält.

7. Verfahren nach einem der vorangehenden Ansprüche, **dadurch gekennzeichnet, daß** zur Isolierung des Glucans ein Massenverhältnis von Hefe zur alkalischen, tensidhaltigen Lösung zwischen 1:10 und 1:400, vorzugsweise zwischen 1:20 und 1:200, eingesetzt wird.

8. Verfahren nach einem der vorangehenden Ansprüche, **dadurch gekennzeichnet, daß** die Hefe während eines ersten Behandlungsschrittes mit der alkalischen, tensidhaltigen Lösung bei einer maximalen Temperatur von 30 °C behandelt wird und daß sich an den ersten Behandlungsschritt ein zweiter Behandlungsschritt anschließt, in dem die Temperatur der alkalischen, tensidhaltigen Lösung auf 50 °C bis 95 °C eingestellt wird.

9. Verfahren nach Anspruch 8, **dadurch gekennzeichnet, daß** der erste Behandlungsschritt in zwei bis acht erste Behandlungsteilschritte aufgeteilt wird, wobei in jedem Behandlungsteilschritt ein fester, Glucan-haltiger Rückstand abgetrennt wird und daß dieser abgetrennte Rückstand jeweils mit einer frischen alkalischen, tensidhaltigen Lösung in Kontakt gebracht wird.

10. Verfahren nach Anspruch 9, **dadurch gekennzeichnet, daß** bei jedem ersten Behandlungsteilschritt das Massenverhältnis von abgetrenntem Glucan-haltigen Rückstand und alkalischer, tensidhaltiger Lösung zwischen 1:1 und 1:40 variiert.

11. Verfahren nach Anspruch 8, **dadurch gekennzeichnet, daß** der zweite Behandlungsschritt in zwei bis acht zweite Behandlungsteilschritte aufgeteilt wird, wobei in jedem Behandlungsteilschritt ein fester Glucan-haltiger Rückstand abgetrennt wird und dieser abgetrennte Rückstand jeweils mit einer frischen alkalischen, tensidhaltigen Lösung in Kontakt gebracht wird.

12. Verfahren nach einem der Ansprüche 9 bis 11, **dadurch gekennzeichnet, daß** als Behandlungszeit für jeden ersten Behandlungsteilschritt und/oder für jeden zweiten Behandlungsteilschritt ein Zeit zwischen 5 Stunden und 48 Stunden ausgewählt wird.

13. Verfahren nach einem der vorangehenden Ansprüche, **dadurch gekennzeichnet, daß** der nach der Behandlung mit der alkalischen, tensidhaltigen Lösung anfallende feste Glucan-haltige Rückstand abgetrennt wird, daß der abgetrennte Rückstand mit Wasser gewaschen wird, daß der gewaschene Rückstand mit dem Alkohol in Kontakt gebracht wird und daß hiernach das gereinigte Glucan aus dem Alkohol abgetrennt wird.

14. Verfahren nach Anspruch 13, **dadurch gekennzeichnet, daß** der gewaschene Rückstand zweimal bis sechsmal mit dem Alkohol in Kontakt gebracht wird.

15. Verfahren nach Anspruch 14, **dadurch gekennzeichnet, daß** nach jedem in Kontakt bringen des Rückstandes mit dem Alkohol der in dem Alkohol unlösliche Glucan-haltige Rückstand abgetrennt wird.

16. Verfahren nach Anspruch 14 oder 15, **dadurch gekennzeichnet, daß** der gewaschene Rückstand jeweils für 5 Stunden bis 48 Stunden bei einer Temperatur zwischen 20 °C und 60 °C mit dem Alkohol in Kontakt gebracht wird.

17. Verfahren nach einem der vorangehenden Ansprüche, **dadurch gekennzeichnet, daß** als Alkohol Isopropanol ausgewählt wird.

## Claims

1. A method for the isolation of glucan from yeast, whereby the yeast is treated with an aqueous alkaline solution, whereby the so isolated solid glucan-containing residue is brought into contact with an alcohol and subsequently the so isolated glucan is dried, **characterized in that** as alkaline solution an alkaline surfactant-containing solution is selected which contains beside an alkali hydroxide and/or an alkali earth hydroxide a surfactant or a surfactant mixture, whereby the concentration of the surfactant or the surfactant mixture varies between 0.05 % by weight and 3 % by weight, and that the glucan-containing residue is brought into contact with a C₁-C₄-alcohol.

2. The method according to claim 1, **characterized in that** the alkaline surfactant-containing solution comprises between 5 % by weight and 0.05 % by weight, preferably between 2 % by weight and 0.01 % by weight, of the alkali hydroxide and/or alkali earth hydroxide.

3. The method according to claim 1 or 2, **characterized in that** the yeast is first of all treated with an alkaline surfactant-containing solution having a higher concentration of alkali hydroxide and/or alkali earth hydroxide and that subsequently the concentration of the alkali hydroxide and/or alkali earth hydroxide is reduced during the isolation of the glucan.

4. The method according to one of the preceding claims, **characterized in that** the alkaline surfactant-containing solution contains as surfactant an anionic and/or a nonionic surfactant.

5. The method according to claim 4, **characterized in that** the alkaline surfactant-containing solution contains as surfactant an alkali salt of an alkyl sulphate and/or an alkali salt of an alkyl sulphonate.

6. The method according to claim 4 or 5, **characterized in that** the alkaline surfactant-containing solution comprises as surfactant a sodium lauryl sulphate.

7. The method according to one of the preceding claims, **characterized in that** for the isolation of the glucan a mass ratio of yeast to the alkaline surfactant-containing solution of between 1:10 and 1:400, preferably of between 1:20 and 1:200, is used.

8. The method according to one of the preceding claims, **characterized in that** during a first treatment step the yeast is treated with the alkaline surfactant-containing solution at a maximum temperature of 30 °C and that after the first treatment step a second treatment step follows in which the temperature of the alkaline surfactant-containing solution is set to between 50 °C and 95 °C.

9. The method according to claim 8, **characterized in that** the first treatment step is divided into two to eight first partial treatment steps, whereby in each partial treatment step a solid glucan-containing residue is separated and that this separated residue is brought into contact with a fresh alkaline surfactant-containing solution.

10. The method according to claim 9, **characterized in that** in each first partial treatment step the mass ratio of the separated glucan-containing residue to the alkaline surfactant-containing solution varies between 1:1 and 1:40.

11. The method according to claim 8, **characterized in that** the second treatment step is divided into two to eight second partial treatment steps, whereby in each partial treatment step a solid glucan-containing residue is separated and that this separated residue is brought into contact with a fresh alkaline surfactant-containing solution.

12. The method according to one of the claims 9 to 11, **characterized in that** a time between 5 hours and 48 hours is selected as treatment time for each first partial treatment step and/or for each second partial treatment step.

13. The method according to one of the preceding claims, **characterized in that** the solid glucan-containing residue is separated after the treatment with the alkaline surfactant-containing solution, that the separated residue is washed with water, that the washed residue is brought into contact with the alcohol and that hereafter the purified glucan is isolated from the alcohol.

14. The method according to claim 13, **characterized in that** the washed residue is brought into contact two to six times with the alcohol.

15. The method according to claim 14, **characterized in that** the glucan-containing residue being insoluble in the alcohol is separated after each contact of the residue with the alcohol.

16. The method according to claim 14 or 15, **characterized in that** the washed residue is brought into contact with the alcohol for 5 hours to 48 hours at a temperature of between 20°C and 60 °C.

17. The method according to one of the preceding claims, **characterized in that** isopropanol is selected as alcohol.

## Revendications

1. Procédé d'isolement de glucane à partir de levure, dans lequel la levure est traitée par une solution aqueuse alcaline, le résidu solide contenant du glucane ainsi obtenu est mis en contact avec un alcool et le glucane ainsi isolé est séché, **caractérisé en ce que** l'on choisit comme solution alcaline une solution alcaline contenant un tensioactif et qui, outre un hydroxyde de métal alcalin et / ou un hydroxyde de métal alcalino-terreux, contient un tensioactif ou un mélange de tensioactifs, la concentration du tensioactif ou du mélange de tensioactifs se situant entre 0,05 % en poids et 3 % en poids, et que le résidu contenant du glucane est mis en contact avec un alcool en C₁ à C₄.

2. Procédé selon la revendication 1, **caractérisé en ce que** la solution alcaline contenant un tensioactif contient entre 5 % en poids et 0,05 % en poids, de préférence entre 2 % en poids et 0,1 % n poids de l'hydroxyde de métal alcalin et / ou de l'hydroxyde de métal alcalino-terreux.

3. Procédé selon la revendication 1 ou 2, **caractérisé en ce que** la levure est d'abord traitée par une solution alcaline contenant un tensioactif, d'une concentration supérieure à la concentration de l'hydroxyde de métal alcalin et / ou de l'hydroxyde de métal alcalino-terreux, et qu'ensuite la concentration de la solution d'hydroxyde de métal alcalin et / ou de métal alcalino-terreux est diminuée au fur et à mesure de l'isolement du glucane.

4. Procédé selon l'une quelconque des revendications qui précèdent, **caractérisé en ce que** la solution alcaline contenant un tensioactif contient, en tant que tensioactif, un tensioactif anionique et /ou non ionique.

5. Procédé selon la revendication 4, **caractérisé en ce que** la solution alcaline contenant un tensioactif contient, en tant que tensioactif, un sel de métal alcalin d'un sulfate d'alkyle et / ou un sel de métal alcalin d'un sulfonate d'alkyle.

6. Procédé selon la revendication 4 ou 5, **caractérisé en ce que** la solution alcaline contenant un tensioactif contient en tant que tensioactif un laurylsulfate de sodium.

7. Procédé selon l'une quelconque des revendications qui précèdent, **caractérisé en ce que**, pour l'isolement du glucane, on met en oeuvre un rapport massique de la levure à la solution alcaline contenant un tensioactif comprise entre 1 : 10 et 1 : 400, de préférence entre 1 : 20 et 1 : 200.

8. Procédé selon l'une quelconque des revendications qui précèdent, **caractérisé en ce que** la levure, pendant une première étape de traitement, est traitée par la solution alcaline contenant un tensioactif à une température maximale de 30°C et que la première étape de traitement est suivie d'une deuxième étape de traitement dans laquelle la température de la solution alcaline contenant un tensioactif est ajustée à une valeur de 50C° à 95°C.

9. Procédé selon la revendication 8, **caractérisé en ce que** la première étape de traitement est divisée en deux à huit premières étapes partielles de traitement, où dans chaque étape partielle de traitement on sépare un résidu solide contenant du glucane, et que ce résidu séparé est à chaque fois mis en contact avec une solution alcaline fraîche contenant un tensioactif.

10. Procédé selon la revendication 9, **caractérisé en ce que**, dans chaque première étape partielle de traitement, le rapport massique du résidu séparé contenant du glucane à la solution alcaline contenant un tensioactif varie entre 1 : 1 et 1 : 40.

11. Procédé selon la revendication 8, **caractérisé en ce que** la deuxième étape de traitement est divisée en deux à huit deuxièmes étapes partielles de traitement, où dans chaque étape partielle de traitement, on sépare un résidu solide contenant du glucane, et ce résidu séparé est à chaque fois mis en contact avec une solution alcaline fraîche contenant un tensioactif.

12. Procédé selon l'une quelconque des revendications 9 à 11, **caractérisé en ce que** l'on choisit comme temps de traitement pour chaque première étape partielle de traitement et / ou pour chaque deuxième étape partielle de traitement un temps compris entre 5 heures et 48 heures.

13. Procédé selon l'une quelconque des revendications qui précèdent, **caractérisé en ce que** le résidu solide contenant du glucane, formé après le traitement par la solution alcaline contenant un tensioactif , est séparé, que le résidu séparé est lavé à l'eau, que le résidu lavé est mis en contact avec l'alcool et qu'ensuite le glucane purifié est séparé de l'alcool.

14. Procédé selon la revendication 13, **caractérisé en ce que** le résidu lavé est mis en contact deux fois à six fois avec l'alcool.

15. Procédé selon la revendication 14, **caractérisé en ce qu'**après chaque mise en contact du résidu avec l'alcool, le résidu contenant du glucane et insoluble dans l'alcool est séparé.

16. Procédé selon la revendication 14 ou 15, **caractérisé en ce que** le résidu lavé est à chaque fois mis en contact avec l'alcool pendant 5 heures à 48 heures, à une température comprise entre 20°C et 60°C.

17. Procédé selon l'une quelconque des revendications qui précèdent, **caractérisé en ce que** l'on choisit comme alcool de l'isopropanol.
